# EUROPEAN PATENT APPLICATION

(11) **EP 3 992 898 A1**
(43) Date of publication of application: **04.05.2022**
(21) Application number: 20937173.1
(22) Date of filing: 02.09.2020
(51) Int. Cl.: G06Q 50/10, G16H 20/00

(54) **INFANT WAKE-UP ASSISTANCE SYSTEM**

(71) Applicant: First Ascent Inc., Tokyo 104-0061 (JP)
(72) Inventor: HATTORI, Tomoyuki, Tokyo 104-0061 (JP)
(74) Representative: Keil & Schaafhausen Patentanwälte PartGmbB
(86) International application number: PCT/JP2020/033330
(87) International publication number: WO 2022/049683

(57) **Abstract**

An infant wake-up assisting system includes an acquisition unit that acquires a plurality of days worth of sleeping status data concerning time-series changes in sleeping and awakening of an infant, a computation unit that calculates a recommended wake-up date and time for the infant, on the basis of at least daily deviations in the plurality of days worth of sleeping status data, and a generating unit that generates a notification message to provide a notification of the recommended wake-up date and time calculated by the computation unit.

## Description

### Technical Field

The present invention relates to an infant wake-up assisting system.

### Background Art

In Patent Document 1, a computer program is disclosed that provides accurate childcare advice in accordance with developmental stages of sleep of an infant. The developmental stages of sleep are classified according to a magnitude of a sleeping score, and a strength of a 24-hour circadian rhythm is calculated as the sleeping score. According to such a computer program, concerning sleeping of the infant, regular physical and mental rest is provided as the childcare advice.

### Prior Art Documents

### Patent Documents

Patent Document 1: JP 2019-010417 A

### Summary of Invention

### Problem the Invention Aims to Solve

A problem arises in that the caregiver of the infant who has received the childcare advice in accordance with the computer program disclosed in Patent Document 1 may force the infant to sleep in an ideal circadian rhythm.

### Means for Solving the Problem

According to a first aspect of the present invention, an infant wake-up assisting system comprises an acquisition unit configured to acquire a plurality of days worth of sleeping status data concerning time-series changes in sleeping and awakening of an infant, a computation unit configured to calculate a recommended wake-up date and time for the infant, on a basis of at least daily deviations in the plurality of days worth of sleeping status data, and a generating unit configured to generate a notification message to provide a notification of the recommended wake-up date and time calculated by the computation unit.

According to a second aspect of the present invention, in the infant wake-up assisting system according to the first aspect, the sleeping status data preferably includes at least one from among sensor data generated by a sensor detecting states in relation to the sleeping and the awakening of the infant, and input data generated by a user inputting information concerning the sleeping and the awakening of the infant.

According to a third aspect of the present invention, in the infant wake-up assisting system according to the first aspect or the second aspect, the sleeping status data preferably includes data representing at least one from among a falling asleep date and time at which the infant fell asleep, a wake-up date and time at which the infant has awakened, a putting-to-sleep starting date and time at which a caregiver of the infant initiates an action of putting the infant to sleep, and a vocalization of the infant.

According to a fourth aspect of the present invention, in the infant wake-up assisting system according to the third aspect, there is preferably further provided an output device configured to output, via at least one from among a screen and voice, the notification message generated by the generating unit to provide the notification of the recommended wake-up date and time. When the sleeping status data includes data of either one of the falling asleep date and time or the putting-to-sleep starting date and time, the output device outputs the notification message at the either one of the dates and times.

According to a fifth aspect of the present invention, in the infant wake-up assisting system according to any one of the first to fourth aspects, the plurality of days worth of sleeping status data is preferably sleeping status data for at least three days.

According to a sixth aspect of the present invention, in the infant wake-up assisting system according to any one of the first to fifth aspects, there is preferably further provided a wake-up prompting device configured to activate a wake-up prompting operation so as to prompt the infant to wake up by changing a sleeping environment of the infant, and a setting unit configured to set an activation date and time at which the wake-up prompting operation by the wake-up prompting device is to be activated.

According to a seventh aspect of the present invention, in the infant wake-up assisting system according to the sixth aspect, there is preferably further provided an input interface configured to allow input of instruction information to instruct that the activation date and time be set to a date and time corresponding to either one of the recommended wake-up date and time or an arbitrary wake-up date and time. The acquisition unit acquires the instruction information that is input to the input interface. The setting unit sets the activation date and time on a basis of the instruction information acquired by the acquisition unit.

According to an eighth aspect of the present invention, in the infant wake-up assisting system according to the sixth aspect or the seventh aspect, the wake-up prompting device preferably includes at least an LED, and the wake-up prompting operation preferably is an operation that changes the sleeping environment by initiating light emission from the LED at the activation date and time.

According to a ninth aspect of the present invention, in the infant wake-up assisting system according to the eighth aspect, a color temperature of light emitted from the LED, the light emission of which is initiated at the activation date and time, is preferably greater than or equal to 4200 K and less than or equal to 6500 K.

According to a tenth aspect of the present invention, in the infant wake-up assisting system according to the ninth aspect, a brightness of the light emitted from the LED preferably increases with a passage of time from the activation date and time.

According to an eleventh aspect of the present invention, in the infant wake up assisting system according to any one of the first to tenth aspects, the computation unit preferably calculates the recommended wake-up date and time on a basis of the plurality of days worth of sleeping status data, the deviations, and a predetermined target wake-up time.

### Effect of the Invention

According to the present invention, an effect can be obtained in that there is a high possibility that sleep training for an infant can be realized with less of a burden being placed on both the infant and a caregiver of the infant.

### Description of Drawings

FIG. 1 is a diagram showing a configuration of an infant wake-up assisting system according to an embodiment of the present invention;
FIG. 2 is a diagram showing a configuration of an input interface and an output device included in the infant wake-up assisting system, and examples of displayed messages;
FIG. 3 is a diagram showing sleeping status data concerning time-series changes in sleeping and awakening of the infant, and a graph corresponding to the data;
FIG. 4 is a diagram illustrating daily deviations in a plurality of days worth of sleeping status data of the infant;
FIG. 5 is a diagram showing an example of calculating an autocorrelation coefficient of the plurality of days worth of sleeping status data of the infant illustrated in FIG. 4;
FIG. 6 is a diagram showing an example of an infant wake-up assisting process executed by running a computer program in the infant wake-up assisting system;
FIG. 7 is a diagram showing an example of a wake-up prompting operation in which a wake-up prompting device prompts the infant to wake up by changing a sleeping environment of the infant;
FIG. 8 is a diagram illustrating a product supplying mode of the computer program that runs in the infant wake-up assisting system;
FIG. 9 is a diagram showing an exemplary configuration of an input interface and an output device included in an infant wake-up assisting system according to an exemplary modification;
FIG. 10 is a diagram showing an aspect in which vocalization data of the infant is acquired as one type of sleeping status data by an acquisition unit of the infant wake-up assisting system according to an exemplary modification;
FIG. 11 is a diagram showing the respective configurations of an electronic device and a wake-up prompting device constituting the infant wake-up assisting system according to an exemplary modification;
FIG. 12 is a diagram showing configurations of an input interface and an output device included in the electronic device shown in FIG. 11, and examples of displayed content; and
FIG. 13 is a diagram showing a server, an electronic device and a wake-up prompting device constituting the infant wake-up assisting system according to an exemplary modification, and respective configurations thereof.

### Description of Embodiments

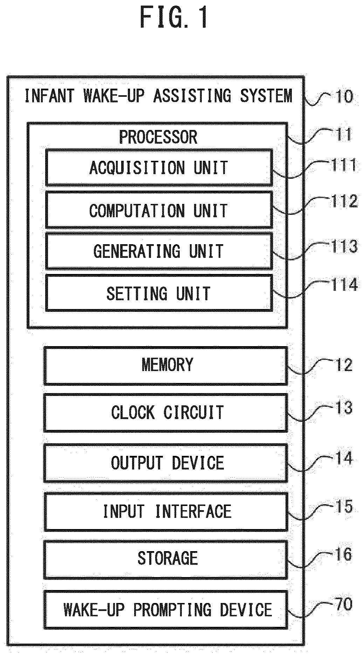

FIG. 1 is a diagram showing a configuration of an infant wake-up assisting system 10 according to an embodiment of the present invention. The infant wake-up assisting system 10 includes a processor 11, a memory 12, a clock circuit 13, an output device 14, an input interface 15, a storage 16, and a wake-up prompting device 70. By activating a computer program stored in the memory 12, the processor 11 logically includes an acquisition unit 111, a computation unit 112, a generating unit 113, and a setting unit 114.

The acquisition unit 111 acquires a plurality of days worth of sleeping status data from the storage 16 in which such data is stored, the data being input via the input interface 15. The storage 16 may be an external storage device of the infant wake-up assisting system 10. The sleeping status data is data concerning time-series changes in sleeping and awakening of the infant, and includes at least one or both of sensor data generated by a biological sensor detecting states in relation to sleeping and awakening of the infant, and user input data generated by a user such as a caregiver of the infant inputting information concerning sleeping and awakening of the infant. The sensor data, for example, is data of brain waves, a body temperature, a heart rate, a respiration, a pulse, and/or a myoelectric potential. In the present embodiment, user input data is used as the sleeping status data. As will be discussed later, by the user operating the input interface 15 when the infant fell asleep and when the infant has awakened, data in which operation signals of the input interface 15 and time data supplied by the clock circuit 13 are associated with each other is stored in the storage 16 as data of falling asleep dates and times at which the infant fell asleep and wake-up dates and times at which the infant has awakened, in other words, as the sleeping status data.

Furthermore, the acquisition unit 111 acquires, via the input interface 15, instruction information to instruct that either one of a recommended wake-up date and time or an arbitrary wake-up date and time be set as an activation date and time at which a wake-up prompting operation by the later-described wake-up prompting device 70 is to be activated. As will be described later, the computation unit 112 calculates the recommended wake-up date and time for the infant, on the basis of at least daily deviations in the sleeping status data acquired by the acquisition unit 111 and stored in the storage 16. The generating unit 113 generates a notification message to provide a notification of the recommended wake-up date and time for the infant as calculated by the computation unit 112. Based on the instruction information acquired by the acquisition unit 111, the setting unit 114 sets the activation date and time at which the wake-up prompting operation by the later-described wake-up prompting device 70 is to be activated.

As was noted previously, the sleeping status data is input to the input interface 15. The sleeping status data thus input is stored in the storage 16. The output device 14 outputs, by way of a screen display and/or voice transmission, the notification message generated by the generating unit 113. In the present embodiment, the output device 14 outputs the notification message by way of a screen display.

At the activation date and time set by the setting unit 114, the wake-up prompting device 70 activates the wake-up prompting operation which prompts the infant to wake up, by changing a sleeping environment of the infant. The sleeping environment is made up from light, sound, wind, temperature and humidity, and/or vibrations, and these undergo a variation in accordance with light emission, ringing, ventilation, control of temperature and humidity, and/or vibrations, respectively. In the present embodiment, the wake-up prompting device 70 includes at least an LED. The wake-up prompting operation activated by the wake-up prompting device 70 is an operation of changing the sleeping environment for the infant by initiating light emission from the LED at the activation date and time set by the setting unit 114.

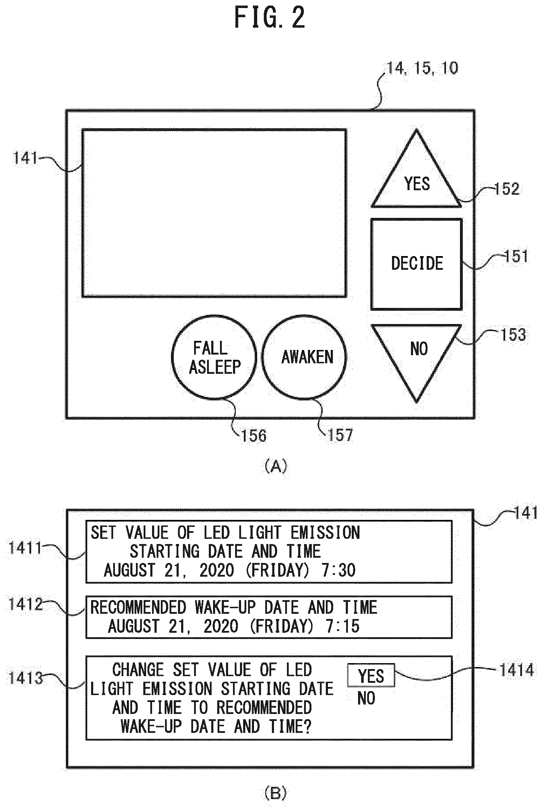

FIG. 2 is a diagram showing a configuration of the input interface 15 and the output device 14 included in the infant wake-up assisting system 10, and examples of displayed messages. In the example shown in FIG. 2(A), the output device 14 included in the infant wake-up assisting system 10 is a touch panel 141 that is configured integrally with the input interface 15, and furthermore, operation buttons 151, 152, 153, 156, and 157 are provided as the input interface 15 separately from the touch panel 141.

In the example shown in FIG. 2(B), notification messages 1411, 1412, and 1413 are displayed on the touch panel 141. The notification message 1411 indicates that a set value of an LED light emission starting date and time, which is set in advance by the user, is 7:30 on August 21, 2020 (Friday). The wake-up prompting operation which prompts the infant to wake up is scheduled to be activated by the LED starting emitting light at the date and time that has been set in this manner, and by the light emitted from the LED being irradiated onto the infant.

The notification message 1412 is generated by the generating unit 113 of the processor 11, and indicates that the recommended wake-up date and time for the infant is 7:15 on August 21, 2020 (Friday). The notification message 1413 indicates an inquiry as to whether or not to change the set value of the LED light emission starting date and time to the recommended wake-up date and time, and options for providing an answer to such an inquiry. By pressing and operating the button 152 or the button 153, the user aligns a rectangular frame 1414 with either one of the answer options of "YES" and "NO", and presses the button 151 to decide upon the answer. When the answer "YES" is selected, the set value of the LED light emission starting date and time is changed from the initial value of 7:30 on August 21, 2020 (Friday) to 7:15 on August 21, 2020 (Friday), which is the recommended wake-up date and time.

Moreover, the set value of the LED light emission starting date and time may be set to a date and time which is earlier than the recommended wake-up date and time by a predetermined time period T. Such a setting is not premised on the infant waking up at the same time that the LED light emission starts, but rather, is based on a concept that the date and time after the predetermined time period T has elapsed from starting of the LED light emission is regarded as the recommended wake-up date and time for the infant.

By pressing the buttons 156 and 157, respectively, the user can input information concerning the sleeping and awakening of the infant. When the user presses the button 156 at a timing at which the infant falls asleep, an operation signal is generated, and data in which the operation signal is associated with the time data supplied by the clock circuit 13 is generated, and such data is input as data of the falling asleep date and time at which the infant fell asleep. When the user presses the button 157 at a timing at which the infant has awakened, an operation signal is generated, and data in which the operation signal is associated with the time data supplied by the clock circuit 13 is generated, and such data is input as data of the wake-up date and time at which the infant has awakened.

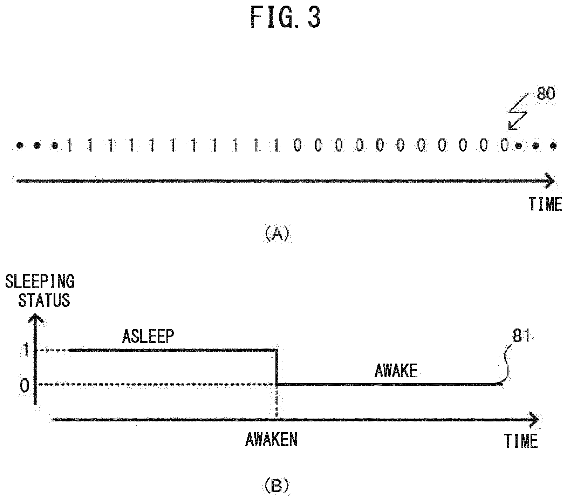

FIG. 3 is a diagram showing sleeping status data 80 concerning time-series changes in sleeping and awakening of the infant, and a graph 81 corresponding to the data. After the time at which the aforementioned button 156 is pressed by the user, the infant is sleeping, and thereafter, after the time at which the aforementioned button 157 is pressed by the user, the infant is awake. If the value of a sleeping status flag when the infant is sleeping is represented by 1, and the value of the sleeping status flag when the infant is awake is represented by 0, respectively, then as shown in FIG. 3 (A), the sleeping status data 80 is represented by arrangement of the sleeping status flag values 0 and 1 in chronological order, and the infant wakes up at a time at which the value of the sleeping status flag changes from 1 to 0. The sleeping status data 80 which is obtained in this manner is stored in the storage 16 as described above.

FIG. 3(B) shows the sleeping status data 80 in the form of the graph 81. The graph 81 is represented on a coordinate plane in which the sleeping status, which has a value of 0 corresponding to awakening or a value of 1 corresponding to sleeping, is indicated on the vertical axis, and time is indicated on the horizontal axis. In the graph 81, the time at which the sleeping status value changes from 1 to 0 represents the wake-up date and time of the infant.

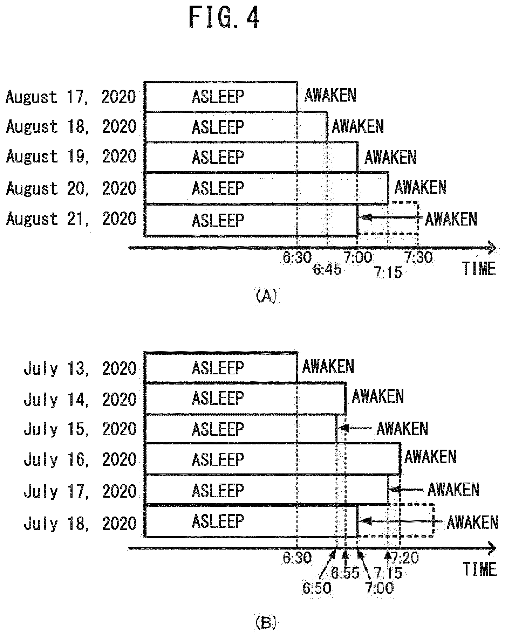

FIG. 4 is a diagram illustrating daily deviations in a plurality of days worth of the sleeping status data 80 of the infant. According to the example shown in FIG. 4(A), an infant has awakened at 6:30 in the morning on August 17, 2020. The infant has awakened at 6:45 in the morning on the next day of August 18th, and the wake-up time is fifteen minutes later than on the previous day. The infant has awakened at 7:00 in the morning on the further next day of August 19th, and the wake-up time is a further fifteen minutes later than on the previous day. The infant has awakened at 7:15 in the morning on the further next day of August 20th, and the wake-up time is a further fifteen minutes later than on the previous day. A deviation occurs such that the wake-up time of the infant becomes fifteen minutes later every day. More specifically, in the wake-up time of the infant, a deviation of fifteen minutes occurs every day, and if such a tendency continues, there is a possibility that the infant will wake up at 7:30 in the morning on August 21st, and the wake-up time will become a further fifteen minutes later than on the previous day.

It is known that the circadian rhythm of human beings is slightly longer than 24 hours, and in the case of infants, if no intervention is taken by a caregiver or the like, a phenomenon referred to as free running, in which such a deviation from the 24-hour cycle is repeated, can occur as in the example shown in FIG. 4(A). If such free running continues, there is a possibility that the infant may experience a daily existence in which day and night are reversed, which places a burden on both the infant and the caregiver. Accordingly, it is preferable for such a tendency of free running to be detected at an early stage, and for a correction in the wake-up time of the infant to be prompted.

In the example shown in FIG. 4(A), the days from August 17, 2020 to August 21, 2020 are designated as first to fifth days, respectively, and a description will be given of a first calculation process of calculating the recommended wake-up date and time for the infant on the fifth day, which is carried out by the computation unit 112 of the processor 11 on the basis of the sleeping status data 80. It is assumed that a target wake-up time of the infant at 7:00 is set initially by a user such as a caregiver or the like. At this time, the wake-up time of the infant on the fourth day, which is one day before, or in other words, the previous wake-up time is 7:15, which is a time after the target wake-up time of 7:00. In such a case, a determination is made as to whether or not a deviation in the wake-up time over the previous four days, namely, the first to fourth days, has a tendency of continuously occurring.

The wake-up time of the infant on the second day is fifteen minutes later than on the first day, the wake-up time of the infant on the third day is thirty minutes later than on the first day, and the wake-up time of the infant on the fourth day is forty-five minutes later than on the first day, and the daily deviation in the wake-up time over the past four days is occurring in a direction of being delayed by fifteen minutes three times continuously. Based on the fact that the daily deviation in the wake-up time occurs three times continuously, the computation unit 112 determines that a deviation in the wake-up time over the past four days has a tendency of continuously occurring, and calculates the recommended wake-up date and time for the infant as being 7:00 on August 21st that is advanced by fifteen minutes from the wake-up time of the infant on August 20th, which is one day prior to August 21st, or in other words, the previous wake-up time of 7:15, so as to be closer to the target wake-up time of 7:00.

The amount of advanced time is not limited to being fifteen minutes, and may be another length of time. For example, taking the amount of advanced time on August 21st as being eight minutes, the recommended wake-up date and time may be 7:07 on August 21st, and on the next day of August 22, taking the amount of further advanced time as being eight minutes, the recommended wake-up date and time may be 6:59 on August 22nd. Further, based on the fact that the daily deviation in the wake-up time over the past three days occurs two times continuously, the computation unit 112 may determine that a deviation in the wake-up time has a tendency of continuously occurring.

According to the example shown in FIG. 4(B), the infant has awakened at 6:30 in the morning on July 13, 2020. The infant has awakened at 6:55 in the morning on the next day of July 14th, and the wake-up time is twenty-five minutes later than on the previous day. The infant has awakened at 6:50 in the morning on the further next day of July 15th, and the wake-up time is five minutes earlier than on the previous day. The infant has awakened at 7:20 in the morning on the further next day of July 16th, and the wake-up time is twenty-five minutes later than on the previous day. The infant has awakened at 7:15 in the morning on the further next day of July 17th, and the wake-up time is five minutes earlier than on the previous day. In the example shown in FIG. 4 (B), unlike the example shown in FIG. 4 (A), although the daily deviation in the wake-up time does not occur three times continuously, a deviation in the wake-up time of the infant as a whole over the past five days has a tendency of continuously occurring. If such a tendency continues, there is a possibility that on July 18th, the infant will wake up at a time significantly after the target wake-up time of 7:00.

In the example shown in FIG. 4(B), the days from July 13, 2020 to July 18, 2020 are designated as first to sixth days, respectively, and a description will be given of a second calculation process of calculating the recommended wake-up date and time for the infant on the sixth day, which is carried out by the computation unit 112 of the processor 11 on the basis of the sleeping status data 80. Moreover, it is assumed that the target wake-up time of the infant at 7:00 is set initially in advance. At this time, the wake-up time of the infant on the fifth day, which is one day before, or in other words, the previous wake-up time is 7:15, which is a time after the target wake-up time of 7:00. In such a case, a determination is made as to whether or not a deviation in the wake-up time over the previous five days, namely, the first to fifth days, has a tendency of continuously occurring.

Based on the wake-up times over the past five days, a moving average value of the wake-up times of adjacent two days can be calculated. The moving average value between the wake-up time of 6:30 on the first day and the wake-up time of 6:55 on the second day is around 6:43. This is regarded as a first moving average value of the wake-up time. A first moving average window corresponding to the first moving average value includes the first day and the second day. The moving average value between the wake-up time of 6:55 on the second day and the wake-up time of 6:50 on the third day is around 6:53. This is regarded as a second moving average value of the wake-up time. A second moving average window corresponding to the second moving average value includes the second day and the third day. A deviation of one day exists between the first moving average window corresponding to the first moving average value, and the second moving average window corresponding to the second moving average value.

The moving average value between the wake-up time of 6:50 on the third day and the wake-up time of 7:20 on the fourth day is 7:05. This is regarded as a third moving average value of the wake-up time. A third moving average window corresponding to the third moving average value includes the third day and the fourth day. A deviation of one day exists between the second moving average window corresponding to the second moving average value, and the third moving average window corresponding to the third moving average value. The moving average value between the wake-up time of 7:20 on the fourth day and the wake-up time of 7:15 on the fifth day is around 7:23. This is regarded as a fourth moving average value of the wake-up time. A fourth moving average window corresponding to the fourth moving average value includes the fourth day and the fifth day. A deviation of one day exists between the third moving average window corresponding to the third moving average value, and the fourth moving average window corresponding to the fourth moving average value.

The second moving average value of the wake-up time is about ten minutes later than the first moving average value, the third moving average value of the wake-up time is about twelve minutes later than the second moving average value, and the fourth moving average value of the wake-up time is about eighteen minutes later than the third moving average value. According to the results of the moving average calculations made four times over the past five days, the daily deviation in the moving average value of the wake-up time is occurring in a direction of being delayed three times continuously. Based on the fact that the daily deviation in the moving average value of the wake-up time occurs three times continuously, the computation unit 112 determines that a deviation in the wake-up time over the past five days has a tendency of continuously occurring, and calculates the recommended wake-up date and time for the infant as being 7:00 on July 18th that is advanced by fifteen minutes from the wake-up time of the infant on July 17th, which is one day before, or in other words, the previous wake-up time of 7:15, so as to be closer to the target wake-up time of 7:00.

The amount of advanced time is not limited to being fifteen minutes, and may be another length of time. For example, taking the amount of advanced time on July 18th as being eight minutes, the recommended wake-up date and time may be 7:07 on July 18th, and on the next day of July 19th, taking the amount of further advanced time as being eight minutes, the recommended wake-up date and time may be 6:59 on July 19th. Further, based on the fact that the daily deviation in the wake-up time over the past four days occurs two times continuously, the computation unit 112 may determine that a deviation in the wake-up time has a tendency of continuously occurring.

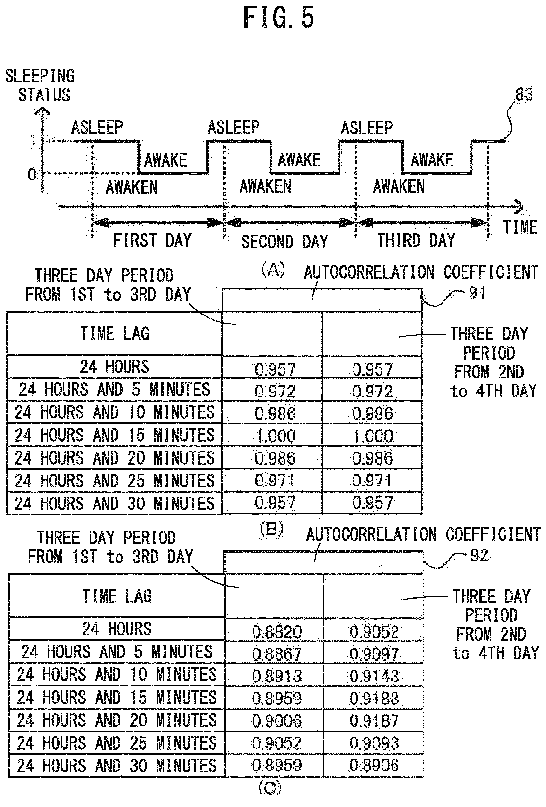

FIG. 5 is a diagram showing an example of calculating an autocorrelation coefficient of the plurality of days worth of the sleeping status data 80 of the infant illustrated in FIG. 4. With reference to FIG. 5, a third calculation process of calculating the recommended wake-up date and time for the infant on the fifth day shown in FIG. 4 will be described. The third calculation process of calculating the recommended wake-up date and time is performed by the computation unit 112 of the processor 11, using the autocorrelation coefficient of the sleeping status data 80. FIG. 5(A) shows a graph 83 corresponding to the sleeping status data 80 concerning time-series changes in sleeping and awakening of the infant from the first day to the third day. From the first day to the third day, if the date and time at which the infant fell asleep and the date and time at which the infant has awakened are always constant, when the autocorrelation coefficient in which a time lag of the sleeping status data 80 is set to one day (24 hours) is calculated, the value of the autocorrelation coefficient becomes 1. If the time lag is increased little by little, the value of the autocorrelation coefficient becomes a value smaller than 1. According to the present embodiment, the autocorrelation coefficient of three consecutive days worth of the sleeping status data 80 is calculated.

FIG. 5(B) shows a table 91 in which there are listed the results of having calculated the autocorrelation coefficient of three consecutive days worth of the sleeping status data 80 from the first day to the third day, and the autocorrelation coefficient of three consecutive days worth of the sleeping status data 80 from the second day to the fourth day, based on the four days worth of the sleeping status data 80 of the infant from August 17, 2020 to August 20, 2020 corresponding to FIG. 4(A). In the table 91, there are shown results in which the respective autocorrelation coefficients are calculated for the time lag that is increased from one day, or in other words, from 24 hours, by five minutes until reaching 24 hours and 30 minutes.

According to the table 91, the values of the autocorrelation coefficient over a three day period from the first day to the third day are 0.957 when the time lag is 24 hours, 0.972 when the time lag is 24 hours and 5 minutes, 0.986 when the time lag is 24 hours and 10 minutes, 1.000 when the time lag is 24 hours and 15 minutes, 0.986 when the time lag is 24 hours and 20 minutes, 0.971 when the time lag is 24 hours and 25 minutes, and 0.957 when the time lag is 24 hours and 30 minutes.

According to the table 91, the values of the autocorrelation coefficient over a three day period from the second day to the fourth day are 0.957 when the time lag is 24 hours, 0.972 when the time lag is 24 hours and 5 minutes, 0.986 when the time lag is 24 hours and 10 minutes, 1.000 when the time lag is 24 hours and 15 minutes, 0.986 when the time lag is 24 hours and 20 minutes, 0.971 when the time lag is 24 hours and 25 minutes, and 0.957 when the time lag is 24 hours and 30 minutes.

According to the table 91, the values of the autocorrelation coefficient over the three day period from the first day to the third day, and the values of the autocorrelation coefficient over the three day period from the second day to the fourth day both have a maximum value of 1.000 when the time lag is 24 hours and 15 minutes, which is more than one day, or in other words, more than 24 hours. Based on the fact that the time lag at which the value of the autocorrelation coefficient has the maximum value continues to have a value greater than 24 hours, the computation unit 112 determines that the deviation in the wake-up time over the past four days has a tendency of continuously occurring, and calculates the recommended wake up date and time for the infant on the fifth day so as to be closer to the target wake-up time of 7:00. The time lag at which the value of the autocorrelation coefficient from the first day to the third day and the value of the autocorrelation coefficient from the second day to the fourth day have the maximum value is 24 hours and 15 minutes two times continuously, and a deviation in the wake-up time of fifteen minutes each per day occurs continuously. Taking this into consideration, for example, due to being advanced by fifteen minutes from 7:15 on the fourth day of August 20th, the recommended wake-up date and time is calculated as being 7:00 on August 21st.

FIG. 5(C) shows a table 92 in which there are listed the results of having calculated the autocorrelation coefficient of three consecutive days worth of the sleeping status data 80 from the first day to the third day, and the autocorrelation coefficient of three consecutive days worth of the sleeping status data 80 from the second day to the fourth day, based on the four days worth of the sleeping status data 80 of the infant from July 13, 2020 to July 16, 2020 corresponding to FIG. 4(B). In the table 92, there are shown results in which the respective autocorrelation coefficients are calculated for the time lag that is increased from one day, or in other words, from 24 hours, by five minutes until reaching 24 hours and 30 minutes.

According to the table 92, the values of the autocorrelation coefficient over a three day period from the first day to the third day are 0.8820 when the time lag is 24 hours, 0.8867 when the time lag is 24 hours and 5 minutes, 0.8913 when the time lag is 24 hours and 10 minutes, 0.8959 when the time lag is 24 hours and 15 minutes, 0.9006 when the time lag is 24 hours and 20 minutes, 0.9052 when the time lag is 24 hours and 25 minutes, and 0.8959 when the time lag is 24 hours and 30 minutes.

According to the table 92, the values of the autocorrelation coefficient over a three day period from the second day to the fourth day are 0.9052 when the time lag is 24 hours, 0.9097 when the time lag is 24 hours and 5 minutes, 0.9143 when the time lag is 24 hours and 10 minutes, 0.9188 when the time lag is 24 hours and 15 minutes, 0.9187 when the time lag is 24 hours and 20 minutes, 0.9093 when the time lag is 24 hours and 25 minutes, and 0.8906 when the time lag is 24 hours and 30 minutes.

According to the table 92, the values of the autocorrelation coefficient over the three day period from the first day to the third day have a maximum value of 0.9052 when the time lag is 24 hours and 25 minutes, which is more than one day, or in other words, more than 24 hours. The values of the autocorrelation coefficient over the three day period from the second day to the fourth day have a maximum value of 0.9188 when the time lag is 24 hours and 15 minutes, which is more than one day, or in other words, more than 24 hours. Based on the fact that the time lag at which the value of the autocorrelation coefficient has the maximum value continues to have a value greater than 24 hours, the computation unit 112 determines that the deviation in the wake-up time over the past four days has a tendency of continuously occurring, and calculates the recommended wake up date and time for the infant on the fifth day so as to be closer to the target wake-up time of 7:00. The time lags at which the value of the autocorrelation coefficient from the first day to the third day and the value of the autocorrelation coefficient from the second day to the fourth day have the maximum value are 24 hours and 25 minutes and 24 hours and 15 minutes, respectively, and on average, a deviation in the wake-up time of twenty minutes each per day occurs continuously. Taking this into consideration, for example, due to being advanced by twenty minutes from 7:20 on the fourth day of July 16th, the recommended wake-up date and time is calculated as being 7:00 on July 17th.

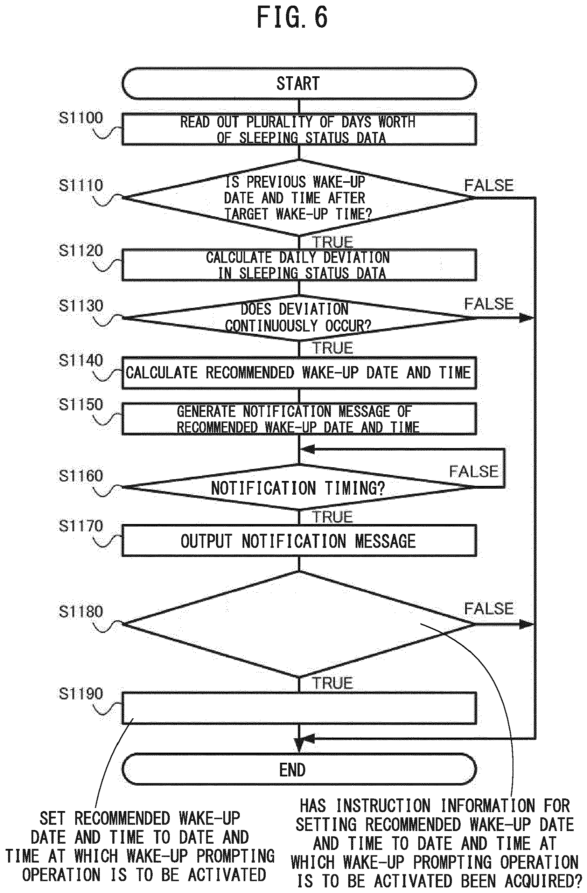

FIG. 6 is a diagram showing an example of an infant wake-up assisting process executed by running a computer program in the infant wake-up assisting system 10. The processor 11 of the infant wake-up assisting system 10 activates the computer program that is stored in the memory 12, and executes the infant wake-up assisting process shown in FIG. 6, thereby functioning as the acquisition unit 111, the computation unit 112, the generating unit 113, and the setting unit 114. In the present embodiment, the infant wake-up assisting process is executed, for example, at a timing at which the aforementioned set value of the LED light emission starting date and time is set by the user, or at a timing at which the user has input information indicating that the infant has awakened by pressing the button 157.

When the infant wake-up assisting process shown in FIG. 6 is initiated, the computation unit 112 of the processor 11 reads out, from the storage 16, a plurality of days worth of the sleeping status data of the infant in step S1100. The plurality of days worth of the sleeping status data of the infant, which is acquired daily by the acquisition unit 111 of the processor 11 via the input interface 15, is stored in the storage 16.

In step S1110, the computation unit 112 determines whether or not a previous wake-up date and time for the infant is a time after the target wake-up time. It should be noted that, instead of the previous wake-up date and time for the infant, or together with the previous wake-up date and time for the infant, a wake-up date and time that is earlier than the previous wake-up date and time for the infant may be used. The target wake-up time is set in advance as described above, and for example, is 7:00. In the case of having obtained a negative determination, the infant wake-up assisting process is brought to an end. In the case of having obtained an affirmative determination, the infant wake-up assisting process proceeds to step S1120.

In step S1120, the computation unit 112 calculates the daily deviations in the plurality of days worth of sleeping status data of the infant. In step S1130, the computation unit 112 determines whether or not the daily deviations in the plurality of days worth of the sleeping status data of the infant occur continuously, for example, three times continuously. In the case of having obtained a negative determination, the infant wake-up assisting process is brought to an end. In the case of having obtained an affirmative determination, the infant wake-up assisting process proceeds to step S1140.

In step S1140, the computation unit 112 calculates the recommended wake-up date and time for the infant, in a manner so that the wake-up time of the infant becomes closer to the target wake-up time than the previous wake-up time. In step S1150, the generating unit 113 of the processor 11 generates the notification message 1412 to provide a notification of the recommended wake-up date and time for the infant as calculated by the computation unit 112.

In step S1160, the generating unit 113 determines whether or not a notification timing for outputting the notification message 1412 to the output device 14 has arrived. The notification timing, for example, is a timing at which the user has input information indicating that the infant has fallen asleep by pressing the button 156 at the date and time at which the infant has fallen asleep. When the user presses the button 156 at a timing at which the infant falls asleep, an operation signal is generated, and data of the date and time at which the infant fell asleep, in which the operation signal is associated with the time data supplied by the clock circuit 13, is generated, and by the generating unit 113 detecting that such data has been acquired as the sleeping status data 80 by the acquisition unit 111, an affirmative determination is made that the notification timing has arrived, whereupon the infant wake-up assisting process proceeds to step S1170. In the case of a negative determination, the determination process of step S1160 is repeated until an affirmative determination is obtained.

In step S1170, the generating unit 113 causes the output device 14 to output the notification message 1412. In the present embodiment, as shown in FIG. 2(B), the output device 14 outputs the notification message 1412 via a screen displayed on the touch panel 141. The output device 14 may also output the notification message 1412 via a voice emitted from a speaker. The generating unit 113 causes the output device 14 to output the notification message 1412 to provide a notification of the recommended wake-up date and time for the infant, together with the notification message 1411 to provide a notification of the set value of the activation date and time at which the wake-up prompting operation by the wake-up prompting device 70 is to be activated, which has been set in advance by the user, and the notification message 1413 to provide a notification of an inquiry as to whether or not the recommended wake-up date and time is to be set to the date and time at which the wake-up prompting operation is to be activated. In the present embodiment, the date and time at which the wake-up prompting operation is to be activated, for example, is the LED light emission starting date and time, and in the example shown in FIG. 2(B), the inquiry as to whether or not to change the set value of the LED light emission starting date and time to the recommended wake-up date and time for the infant is displayed as the notification message 1413 on the touch panel 141.

In step S1170, since the notification messages 1411, 1412, and 1413 are output at the arrival of the notification timing in step S1160, the notification messages can be visually recognized by the user who has pressed the button 156. As described above with reference to FIG. 2, by pressing the button 152 or 153 and the button 151, the user can input an answer to the inquiry, as indicated in the notification message 1413, as to whether or not to change the set value of the LED light emission starting date and time to the recommended wake-up date and time for the infant. When the user presses the buttons 152 and 151, instruction information is input to instruct that the set value of the LED light emission starting date and time should be changed to the recommended wake-up date and time for the infant, and when the user presses the buttons 153 and 151, instruction information is input to instruct that the set value of the LED light emission starting date and time is not to be changed, and should remain at the arbitrary wake-up date and time that was set in advance by the user. In this manner, the acquisition unit 111 of the processor 11 acquires, via the input interface 15, instruction information to instruct that either one of the recommended wake-up date and time or the arbitrary wake-up date and time for the infant be set as the activation date and time at which the wake-up prompting operation by the wake-up prompting device 70 is to be activated.

In step S1180, the acquisition unit 111 determines whether or not the instruction information for setting the recommended wake-up date and time for the infant to the date and time at which the wake-up prompting operation is to be activated has been acquired. In the case that instruction information to instruct that an arbitrary wake-up date and time be set to the date and time at which the wake-up prompting operation is to be activated is acquired by the acquisition unit 111, a negative determination is obtained in step S1180, and the infant wake-up assisting process is brought to an end. In the case that the instruction information to instruct that the recommended wake-up date and time for the infant be set to the date and time at which the wake-up prompting operation is to be activated is acquired by the acquisition unit 111, an affirmative determination is obtained in step S1180, and the infant wake-up assisting process proceeds to step S1190.

In step S1190, based on the instruction information acquired by the acquisition unit 111, the setting unit 114 of the processor 11 sets the recommended wake-up date and time for the infant to the activation date and time at which the wake-up prompting operation by the wake-up prompting device 70 is to be activated. When the process of step S1190 is completed, the infant wake-up assisting process is brought to an end.

Moreover, as noted previously, the set value of the LED light emission starting date and time may be set to a date and time which is earlier than the recommended wake-up date and time by the predetermined time period T. In that case, when the instruction information to instruct that the set value of the LED light emission starting date and time be changed to the recommended wake-up date and time for the infant is acquired by the acquisition unit 111, then based on the instruction information, the setting unit 114 sets a date and time, which is earlier than the recommended wake-up date and time for the infant by the predetermined time period T, to the activation date and time at which the wake-up prompting operation by the wake-up prompting device 70 is to be activated.

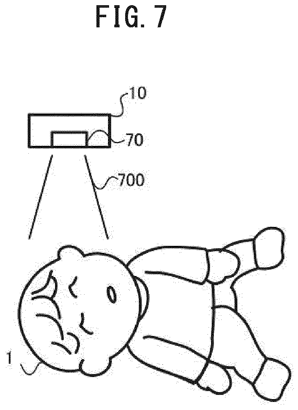

FIG. 7 is a diagram showing an example of a wake-up prompting operation in which the wake-up prompting device 70 prompts an infant 1 to wake up by changing the sleeping environment of the infant 1. The wake-up prompting device 70 that is installed in the infant wake-up assisting system 10 according to the present embodiment includes an LED, and the LED emits light as the wake-up prompting operation which prompts the infant 1 to wake up. By the light emission of the LED being initiated at the date and time at which the wake-up prompting operation is to be activated, light 700 is irradiated onto the infant 1 from the wake-up prompting device 70, whereby the sleeping environment of the infant 1 can be made to change. Insofar as the sleeping environment of the infant 1 is capable of being changed, the wake-up prompting device 70 is not limited to being an LED, and may be a device such as a speaker, a fan, a cooling device, a humidifier, a pressure reducing device, a stimulus generating device, or a vibration device.

When it is taken into consideration that the color temperature of light is disclosed as being related to an effect of inhibiting the production of melatonin in JP 2020-098855 A, it is preferable that the color temperature of the light, which is irradiated onto the infant 1 from the wake-up prompting device 70 of the infant wake-up assisting system 10 in the present embodiment, is on the same order as that of natural morning light during a time period from morning to noon, so as to be capable of prompting the infant 1 to wake up with less of a burden. According to WO 2013/061942 A1, the color temperature of natural light at 7:00 am is 4200 K, and according to JP 2020-087897 A, the color temperature of daylight coloring serving as natural light at noon is 6500 K. Therefore, the color temperature of the light 700 which is irradiated onto the infant 1, and which is emitted from the LED whose light emission is initiated at the date and time at which the wake-up prompting operation is to be activated, is preferably greater than or equal to 4200 K and less than or equal to 6500 K. Further, in imitation of a change in the brightness of natural light in the morning, the brightness of the light 700 emitted from the LED and irradiated onto the infant 1 preferably increases with the passage of time, after the light emission from the LED is initiated at the date and time at which the wake-up prompting operation is to be activated. Moreover, in the case that the date and time at which the wake-up prompting operation is to be activated is set to a date and time which is earlier than the recommended wake-up date and time for the infant 1 by the predetermined time period T, it is also possible to gradually increase the brightness of the LED which has started to emit light at the date and time at which the wake-up prompting operation is to be activated, so that the brightness reaches a maximum brightness value at the recommended wake-up date and time for the infant 1.

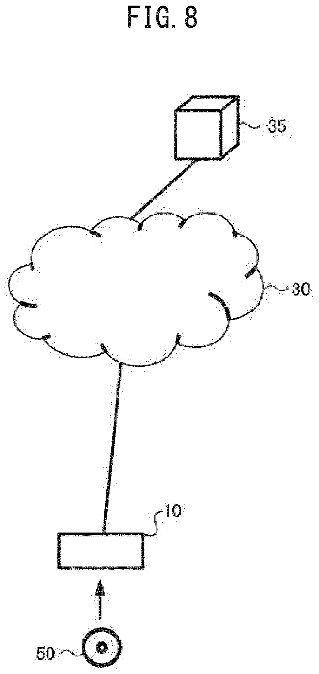

FIG. 8 is a diagram illustrating a product supplying mode of a computer program that runs in the infant wake-up assisting system 10. The computer program that runs in the infant wake-up assisting system 10 can be supplied to the infant wake-up assisting system 10 through a recording medium 50 such as a CD-ROM or a USB flash drive, or through a data signal that flows over a communication network 30 such as the Internet.

A computer program providing server 35 is a server computer that supplies the aforementioned computer program, and stores the computer program in a storage device such as a hard disk or the like. The communication network 30 is the Internet, a wireless LAN, a telephone network, or alternatively, a dedicated line or the like. The computer program providing server 35 reads out the computer program that is stored in the storage device, places it in the form of a data signal on a carrier wave, and transmits the data signal to the infant wake-up assisting system 10 via the communication network 30. In this manner, the computer program can be supplied as a computer-readable computer program product in various formats such as a recording medium, a data signal, or the like.

According to the infant wake-up assisting system 10 in the present embodiment, the following advantageous effects can be obtained.
(1) The infant wake-up assisting system 10 includes the processor 11 containing the acquisition unit 111, the computation unit 112, and the generating unit 113. The acquisition unit 111 acquires the plurality of days worth of the sleeping status data 80 concerning time-series changes in sleeping and awakening of the infant 1. The computation unit 112 calculates the recommended wake-up date and time for the infant 1, on the basis of at least daily deviations in the plurality of days worth of the sleeping status data 80. The generating unit 113 generates the notification message 1412 to provide a notification of the recommended wake-up date and time calculated by the computation unit 112. The notification message of the recommended wake-up date and time is capable of prompting a user such as the caregiver of the infant 1 or the like to correct the wake-up time of the infant 1 prior to a deviation of the sleeping status data 80 being accumulated and becoming large. Accordingly, an effect can be obtained in that there is a high possibility that sleep training for the infant 1 can be realized with less of a burden being placed on both the infant 1 and a caregiver of the infant 1.
(2) In the infant wake-up assisting system 10, the sleeping status data 80 includes at least one from among the sensor data and the input data. The sensor data is generated by the biological sensor detecting states in relation to sleeping and awakening of the infant 1. The input data is data of the falling asleep date and time and the wake-up date and time of the infant 1, which is generated by the user inputting information concerning the sleeping and the awakening of the infant 1 using the buttons 156 and 157 included in the input interface 15. An effect can be obtained in that the sleeping status data 80 can be input easily and accurately.
(3) In the infant wake-up assisting system 10, the sleeping status data 80 includes data representing at least one from among a falling asleep date and time at which the infant 1 fell asleep, and a wake-up date and time at which the infant 1 has awakened. When the sleeping status data 80 includes data representing the wake-up date and time of the infant 1, the recommended wake-up date and time for the infant 1 can be calculated prior to a deviation being accumulated, based on a daily deviation in the wake-up time of a plurality of days in the past. When the sleeping status data 80 includes data representing the falling asleep date and time at which the infant 1 fell asleep and the wake-up date and time at which the infant 1 has awakened, an effect can be obtained in that the recommended wake-up date and time for the infant 1 can be calculated prior to a deviation being accumulated, based on the daily deviation in the wake-up time of a plurality of days in the past determined using the autocorrelation coefficient of the sleeping status data 80.
(4) The infant wake-up assisting system 10 further includes the output device 14 that outputs, via at least one from among a screen displayed on the touch panel 141 and a voice output from the speaker, the notification message 1412 generated by the generating unit 113 to provide a notification of the recommended wake-up date and time. When the sleeping status data 80 includes the data of the date and time of falling asleep of the infant 1, the output device 14 outputs the notification message 1412 at the date and time at which the infant 1 falls asleep. An effect can be obtained in that the user who presses the button 156 at the date and time at which the infant 1 falls asleep can easily visually recognize the notification message 1412 output via the screen displayed on the touch panel 141 which is provided in the vicinity of the button 156, and can know the recommended wake-up date and time for the infant 1.
(5) In the infant wake-up assisting system 10, the plurality of days worth of the sleeping status data 80 is sleeping status data for at least three days. For example, as described with reference to FIG. 4(A), based on the fact that the daily deviation in the wake-up time of the infant 1 over the past three days occurs two times continuously, the computation unit 112 of the processor 11 is capable of determining that the deviation in the wake-up time of the infant 1 has a tendency of continuously occurring. Similarly, in the example shown in FIG. 4(B) or FIG. 5, from the sleeping status data 80 of the infant 1 over the past three days, the computation unit 112 of the processor 11 is capable of determining that the deviation in the wake-up time of the infant 1 has a tendency of continuously occurring. The sleeping status data 80 over a small number of days is suitable in the case that the capacity of the storage 16 is limited, and an effect is obtained in that the computation unit 112 is capable of reducing the computational load when calculating the recommended wake-up date and time for the infant 1.
(6) The infant wake-up assisting system 10 further includes the wake-up prompting device 70 comprising the LED. The processor 11 further includes the setting unit 114. The wake-up prompting device 70 changes the sleeping environment of the infant 1 by irradiating the emitted light 700 of the LED onto the infant 1. In this manner, the wake-up prompting device 70 activates the wake-up prompting operation of initiating light emission from the LED so as to prompt the infant 1 to wake up by changing the sleeping environment of the infant 1. The setting unit 114 sets an activation date and time at which the wake-up prompting operation by the wake-up prompting device 70 is to be activated. Thus, an effect can be obtained in that, without any need for a caregiver of the infant 1 to perform the action by himself/herself of prompting the infant 1 to wake up, the burden on the caregiver can be reduced.
(7) The infant wake-up assisting system 10 further includes the input interface 15 including the buttons 151, 152, and 153 which allow input of instruction information to instruct that the activation date and time at which the wake-up prompting operation by the wake-up prompting device 70 is to be activated be set to a date and time corresponding to either one of the recommended wake-up date and time or an arbitrary wake-up date and time. The acquisition unit 111 of the processor 11 acquires the instruction information that is input to the input interface 15. The setting unit 114 of the processor 11 sets the activation date and time based on the instruction information acquired by the acquisition unit 111. Since the infant wake-up assisting system 10 can be applied to both cases, namely, a case in which sleep training for the infant 1 is to be given priority, and a case in which consideration is given to a caregiver of the infant 1 waking up at a later time, an effect can be obtained in that the burden placed not only on the infant 1, but also on the caregiver of the infant 1 is small.
(8) In the infant wake-up assisting system 10, the wake-up prompting device 70 includes at least the LED. The wake-up prompting operation by the wake-up prompting device 70 is an operation that changes the sleeping environment of the infant 1 by irradiating the emitted light 700 of the LED onto the infant 1, by initiating light emission from the LED at the activation date and time at which the wake-up prompting operation is to be activated. Thus, an effect can be obtained in that the infant 1 is prompted to wake up automatically and safely.
(9) In the infant wake-up assisting system 10, the color temperature of the light 700 emitted from the LED whose light emission is initiated at the activation date and time at which the wake-up prompting operation by the wake-up prompting device 70 is to be activated, is greater than or equal to 4200 K and less than or equal to 6500 K. By irradiation of the light 700 having a color temperature similar to that of natural morning light during a time period from morning to noon, it is possible to obtain an effect in which the infant 1 can be prompted to wake up with less of a burden.
(10) In the infant wake-up assisting system 10, the brightness of the light 700 emitted from the LED increases with the passage of time from the activation date and time at which the wake-up prompting operation by the wake-up prompting device 70 is to be activated. By irradiation of the light 700 in which a change in the brightness of natural light in the morning is imitated, it is possible to obtain an effect in which the infant 1 can be prompted to wake up with less of a burden.
(11) In the infant wake up assisting system 10, the computation unit 112 of the processor 11 calculates the recommended wake-up date and time for the infant 1 on the basis of the plurality of days worth of the sleeping status data 80 including data of a previous wake-up time, at least daily deviations in the plurality of days worth of the sleeping status data 80, and the predetermined target wake-up time of 7:00. By bringing the recommended wake-up date and time in closer proximity to the predetermined target wake-up time of 7:00, it is possible to obtain an effect in which there is a high possibility that sleep training for the infant 1 can be realized with less of a burden.

The following modifications also lie within the scope of the present invention, and one or plurality of such exemplary modifications, which are indicated below, can be combined with the above-described embodiment.
(1) In the above-described embodiment, data of the falling asleep date and time at which the infant 1 fell asleep, and the wake-up date and time at which the infant 1 has awakened are acquired as the sleeping status data 80 by the acquisition unit 111 of the processor 11, and such data are stored in the storage 16. However, the sleeping status data 80 may further include a putting-to-sleep starting date and time at which the caregiver of the infant 1 initiates an action of putting the infant 1 to sleep. The putting-to-sleep starting date and time of the infant 1 is acquired by the acquisition unit 111 and stored in the storage 16, in the same manner as the falling asleep date and time and the wake-up date and time of the infant 1. More specifically, by the user who is the caregiver of the infant 1 operating the input interface 15, data in which operation signals of the input interface 15 and the time data supplied by the clock circuit 13 are associated with each other is stored in the storage 16 as data of the putting-to-sleep starting date and time at which the caregiver of the infant 1 initiates the action of putting the infant 1 to sleep.

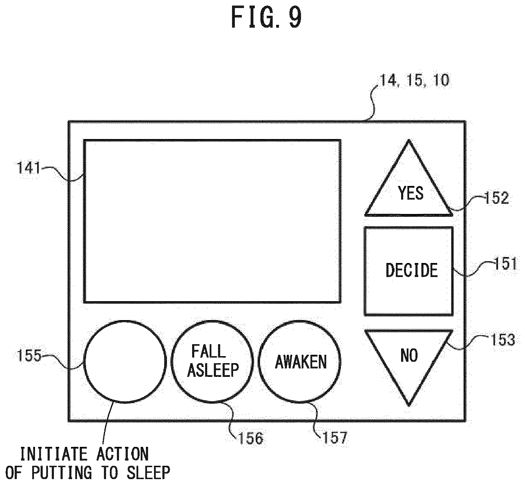

FIG. 9 is a diagram showing an exemplary configuration of the input interface 15 and the output device 14 included in the infant wake-up assisting system 10 according to an exemplary modification. FIG. 9 includes the entire configuration shown in FIG. 2(A), and furthermore, an operation button 155 is provided therein as the input interface 15. In FIG. 9, the structural components having common reference numerals to those shown in FIG. 2(A) are provided with the same functions, and thus, descriptions thereof will be omitted.

By pressing the button 155, a user such as a caregiver or the like can input information concerning the caregiver initiating the action of putting the infant 1 to sleep. When the user presses the button 155 at the timing at which the caregiver initiates the action of putting the infant 1 to sleep, an operation signal is generated, and data in which the operation signal and the time data supplied by the clock circuit 13 are associated with each other is generated. The data is input as data of the putting-to-sleep starting date and time at which the caregiver initiates the action of putting the infant 1 to sleep, and such data is acquired by the acquisition unit 111 of the processor 11. The acquisition unit 111 stores the data of the putting-to-sleep starting date and time in the storage 16.

On the basis of the putting-to-sleep starting date and time at which the caregiver initiates the action of putting the infant 1 to sleep, and the falling asleep date and time at which the infant 1 has fallen asleep, the acquisition unit 111 is capable of obtaining a time period required for the caregiver to put the infant 1 to sleep, namely, a putting-to-sleep required time period. In the case that the putting-to-sleep required time period is longer, for example, than an average value thereof, it may be considered that there is a possibility that a phenomenon is occurring in which the caregiver is attempting to force the infant 1 to go to sleep. In such a case, there is a possibility that the burden placed on the infant 1 is increasing. It is preferable that such a burden can be reduced by correcting the recommended wake-up date and time for the infant 1. For example, there is a possibility that the infant 1 can wake up with less of a burden by performing correction of delaying the recommended wake-up date and time for the infant 1 by a time period obtained as a difference between a previous putting-to-sleep required time period and an average value of the putting-to-sleep required time period in a predetermined interval.

(2) In the above-described embodiment, data of the falling asleep date and time at which the infant 1 fell asleep, and the wake-up date and time at which the infant 1 has awakened are acquired as the sleeping status data 80 by the acquisition unit 111 of the processor 11, and such data are stored in the storage 16. However, the infant 1 may cry at night between falling asleep at night and awakening in the morning, and therefore it is conceivable that the tendency of free running is easily detected by taking into consideration the time period during which such crying at night occurs.

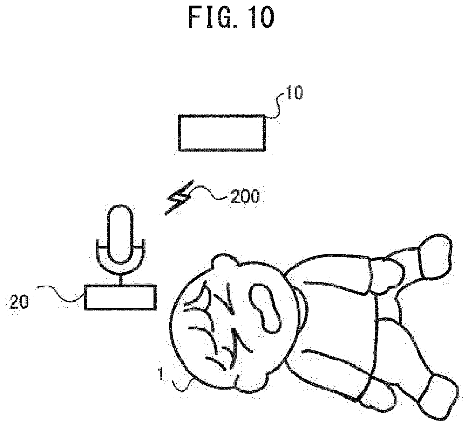

FIG. 10 is a diagram showing an aspect in which vocalization data of the infant 1 is acquired as one type of the sleeping status data 80 by the acquisition unit of the infant wake-up assisting system 10 according to an exemplary modification. A vocalization sensor 20 collects vocalizations of the infant 1 with a microphone, and converts such vocalizations into time-series vocalization data in the form of electrical signals. The time period during which the infant 1 is making a vocalization between the falling asleep date and time and the wake-up date and time is considered to be a time period during which the infant 1 is crying at night. The vocalization sensor 20 outputs the vocalization data to the infant wake-up assisting system 10 via a wireless communication 200 such as Bluetooth (registered trademark) or the like. The vocalization data is acquired by the acquisition unit 111 of the processor 11 via the input interface 15 of the infant wake-up assisting system 10, and is stored in the storage 16 as the sleeping status data 80.

It is conceivable that the tendency of free running is easily detected by correcting the sleeping status data 80 of the infant 1 on the basis of the vocalization data of the infant 1 acquired by the acquisition unit 111, by treating a vocalization time period of the infant 1, in other words, a time period during which the infant 1 is crying at night, as a time period during which the infant is awake. The computation unit 112 calculates the autocorrelation coefficient of the plurality of days worth of the sleeping status data 80 of the infant 1 shown in FIG. 5 by using the sleeping status data 80 of the infant 1 on which such a correction has been made, to thereby calculate the daily deviations in the plurality of days worth of the sleeping status data 80, and the recommended wake-up date and time for the infant 1.

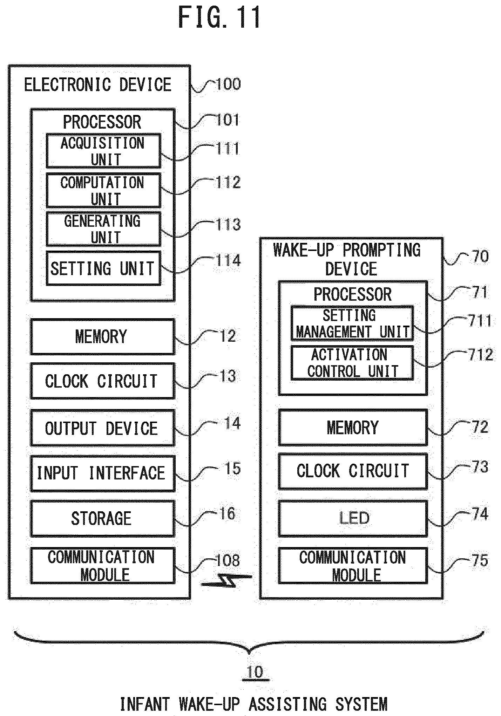

(3) In the above-described embodiment and the exemplary modification, the infant wake-up assisting system 10 is physically configured in the form of one piece of hardware; however, the present invention is not limited to such a configuration. FIG. 11 is a diagram showing the respective configurations of an electronic device 100 and the wake-up prompting device 70 constituting the infant wake-up assisting system 10 according to an exemplary modification. In FIG. 11, the wake-up prompting device 70 is separated in the form of independent hardware from the infant wake-up assisting system 10 shown in FIG. 1, and the remaining functional blocks other than the wake-up prompting device 70 constitute the electronic device 100. The electronic device 100 and the wake-up prompting device 70 are connected to each other via a wireless communication such as Bluetooth (registered trademark) or the like.

The electronic device 100 includes a processor 101, the memory 12, the clock circuit 13, the output device 14, the input interface 15, the storage 16, and a communication module 108. By activating a computer program stored in the memory 12, the processor 101 logically includes the acquisition unit 111, the computation unit 112, the generating unit 113, and the setting unit 114. In FIG. 11, the functions executed by the functional blocks having the same reference numerals as those shown in FIG. 1 are the same functions as described previously, and thus, descriptions of such functions will be omitted. The communication module 108 is used for communicating wirelessly with the aforementioned wake-up prompting device 70.

The wake-up prompting device 70 includes a processor 71, a memory 72, a clock circuit 73, an LED 74, and a communication module 75. By activating a computer program stored in the memory 72, the processor 71 logically includes a setting management unit 711 and an activation control unit 712. The setting management unit 711 acquires and manages, via the communication module 75, the activation date and time at which the wake-up prompting operation by the wake-up prompting device 70 is to be activated, the activation date and time being set by the setting unit 114 included in the processor 101 of the electronic device 100. When the setting management unit 711 detects that the activation date and time at which the wake-up prompting operation by the wake-up prompting device 70 is to be activated has arrived on the basis of the time data supplied by the clock circuit 73, the activation control unit 712 causes the light emission from the LED 74 to be started.

When the user operates the input interface 15 of the electronic device 100 when the infant 1 has awakened, the processor 101 of the electronic device 100 transmits, to the wake-up prompting device 70 via the communication module 108, a signal to provide a notification to the effect that the infant 1 has awakened. In the wake-up prompting device 70, which has received such a signal via the communication module 75, the activation control unit 712 of the processor 71 causes the light emission from the LED 74 to be stopped.

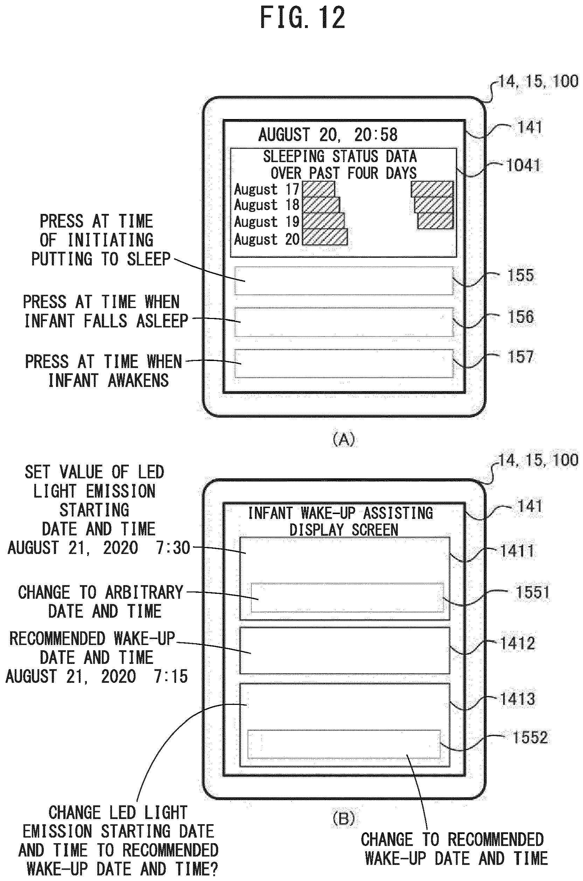

FIG. 12 is a diagram showing configurations of the input interface 15 and the output device 14 included in the electronic device 100 shown in FIG. 11, and examples of displayed content. In FIG. 12, the functions executed by the functional blocks having the same reference numerals as those shown in FIGS. 2 and 9 are the same functions as described previously, and thus, descriptions of such functions will be omitted. In FIG. 12(A), operation buttons 155, 156, and 157 are displayed in the form of images on the touch panel 141. Furthermore, a historical display 1041 of the sleeping status data over the past four days is also provided on the touch panel 141. By referring to the display 1041 of the sleeping status data over the past four days, the user is more likely to notice that a tendency of free running is occurring in the infant 1.

In FIG. 12(B), the notification messages 1411, 1412, and 1413 are displayed on the touch panel 141 in the same manner as in FIGS. 2 and 9. The set value of the LED light emission starting date and time which has been set in advance by the user and notified by the notification message 1411, can be changed to an arbitrary date and time by the user pressing on an input field 1551. In response to an inquiry, as notified by the notification message 1413, as to whether or not to change the set value of the LED light emission starting date and time to the recommended wake-up date and time, in the case of changing, the set value of the LED light emission starting date and time can be changed to the recommended wake-up date and time by the user pressing on an input field 1552.

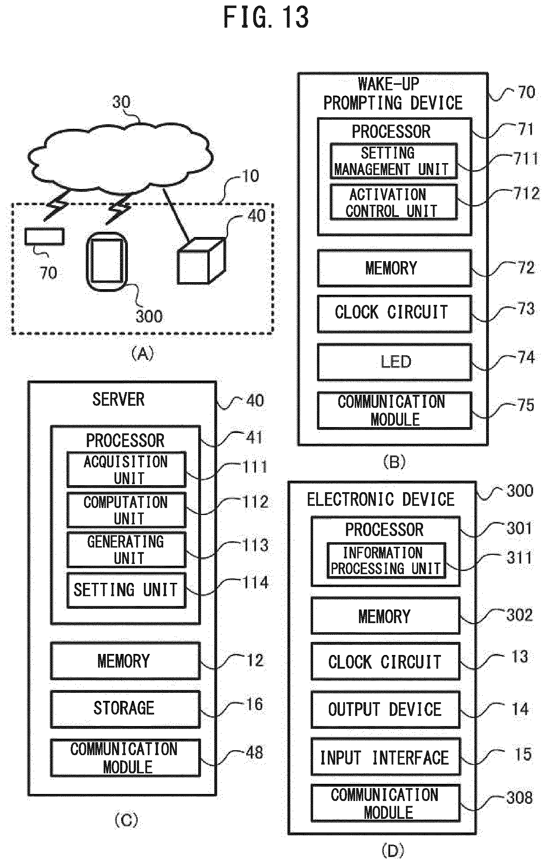

(4) In the above-described embodiment and the exemplary modification, the infant wake-up assisting system 10 is configured in the form of a stand-alone system; however, the present invention is not limited to such a configuration. FIG. 13 is a diagram showing a server 40, an electronic device 300 and the wake-up prompting device 70 constituting the infant wake-up assisting system 10 according to an exemplary modification, and respective configurations thereof. In FIG. 13, the memory 12, the storage 16, the acquisition unit 111, the computation unit 112, the generating unit 113, and the setting unit 114 included in the electronic device 100 shown in FIG. 11 are disposed in the server 40.

In FIG. 13(A), the electronic device 300, the wake-up prompting device 70, and the server 40 are connected to each other via the communication network 30. Since the wake-up prompting device 70 shown in FIG. 13(B) has the same configuration as that of the wake-up prompting device 70 shown in FIG. 11, description thereof will be omitted. In FIG. 13(C), the server 40 includes a processor 41, the memory 12, the storage 16, and a communication module 48. By activating a computer program stored in the memory 12, the processor 41 logically includes the acquisition unit 111, the computation unit 112, the generating unit 113, and the setting unit 114. In FIG. 13(C), the functions executed by the functional blocks having the same reference numerals as those shown in FIGS. 1 and 11 are the same functions as described previously, and thus, descriptions of such functions will be omitted. The communication module 48 is used for communicating with the electronic device 300 and the wake-up prompting device 70 via the communication network 30.

In FIG. 13(D), the electronic device 300 includes a processor 301, a memory 302, the clock circuit 13, the output device 14, the input interface 15, and a communication module 308. By activating a computer program stored in the memory 302, the processor 301 logically includes an information processing unit 311. In FIG. 13(D), the functions executed by the functional blocks having the same reference numerals as those shown in FIGS. 1 and 11 are the same functions as described previously, and thus, descriptions of such functions will be omitted. The communication module 308 is used for communicating with the electronic device 300 and the wake-up prompting device 70 via the communication network 30.

The information processing unit 311 of the processor 301 transmits, to the server 40 via the communication module 308, the plurality of days worth of the sleeping status data 80 input via the input interface 15. By the user operating the input interface 15 when the infant fell asleep and when the infant has awakened, data in which operation signals of the input interface 15 and the time data supplied by the clock circuit 13 are associated with each other is transmitted to the server 40 as data of falling asleep dates and times at which the infant fell asleep and wake-up dates and times at which the infant has awakened, in other words, as the sleeping status data 80. The sleeping status data 80 is acquired by the acquisition unit 111 included in the processor 41 of the server 40, and such data is stored in the storage 16.

The notification messages 1411, 1412, and 1413 generated by the generating unit 113 included in the processor 41 of the server 40 are transmitted to the electronic device 300 via the communication module 48. The notification messages 1411, 1412, and 1413 are acquired by the information processing unit 311 included in the processor 301 of the electronic device 300 via the communication module 308, and are output to the output device 14.

Instruction information to instruct that either one of the recommended wake-up date and time or the arbitrary wake-up date and time be set as the activation date and time at which the wake-up prompting operation by the wake-up prompting device 70 is to be activated is input by the user to the electronic device 300 via the input interface 15. The instruction information thus input is acquired by the acquisition unit 111 included in the processor 41 of the server 40 via the communication module 48. Based on the instruction information acquired by the acquisition unit 111, the setting unit 114 included in the processor 41 sets the activation date and time at which the wake-up prompting operation by the wake-up prompting device 70 is to be activated.

The present invention is not limited to the configurations in the above-described embodiments and exemplary modifications, insofar as the characteristic functions of the present invention are not impaired.

The disclosed content of the following patent documents are incorporated herein by reference.
JP 2020-098855 A
WO 2013/061942 A1
JP 2020-087897 A

### Reference Signs List

1 infant
10 infant wake-up assisting system
11 processor
12 memory
13 clock circuit
14 output device
15 input interface
16 storage
20 vocalization sensor
30 communication network
35 computer program providing server
40 server
41 processor
48 communication module
50 recording medium
70 wake-up prompting device
71 processor
72 memory
73 clock circuit
74 LED
75 communication module
80 sleeping status data
81, 83 graphs
91, 92 tables
100 electronic device
101 processor
108 communication module
111 acquisition unit
112 computation unit
113 generating unit
114 setting unit
141 touch panel
151, 152, 153, 155, 156, 157 buttons
200 wireless communication
300 electronic device
301 processor
302 memory
308 communication module
311 information processing unit
700 light
711 setting management unit
712 activation control unit
1041 historical display
1411, 1412, 1413 notification messages
1414 rectangular frame
1551, 1552 input fields

## Claims

1. An infant wake-up assisting system, comprising:
an acquisition unit configured to acquire a plurality of days worth of sleeping status data concerning time-series changes in sleeping and awakening of an infant;
a computation unit configured to calculate a recommended wake-up date and time for the infant, on a basis of at least daily deviations in the plurality of days worth of sleeping status data; and
a generating unit configured to generate a notification message to provide a notification of the recommended wake-up date and time calculated by the computation unit.

2. The infant wake-up assisting system according to claim 1, wherein the sleeping status data includes at least one from among sensor data generated by a sensor detecting states in relation to the sleeping and the awakening of the infant, and input data generated by a user inputting information concerning the sleeping and the awakening of the infant.

3. The infant wake-up assisting system according to claim 1 or 2, wherein the sleeping status data includes data representing at least one from among a falling asleep date and time at which the infant fell asleep, a wake-up date and time at which the infant has awakened, a putting-to-sleep starting date and time at which a caregiver of the infant initiates an action of putting the infant to sleep, and a vocalization of the infant.

4. The infant wake-up assisting system according to claim 3, further comprising:
an output device configured to output, via at least one from among a screen and voice, the notification message generated by the generating unit to provide the notification of the recommended wake-up date and time,
wherein, when the sleeping status data includes data of either one of the falling asleep date and time or the putting-to-sleep starting date and time, the output device outputs the notification message at the either one of the dates and times.

5. The infant wake-up assisting system according to any one of claims 1 to 4, wherein the plurality of days worth of sleeping status data is sleeping status data for at least three days.

6. The infant wake-up assisting system according to any one of claims 1 to 5, further comprising:
a wake-up prompting device configured to activate a wake-up prompting operation so as to prompt the infant to wake up by changing a sleeping environment of the infant; and
a setting unit configured to set an activation date and time at which the wake-up prompting operation by the wake-up prompting device is to be activated.

7. The infant wake-up assisting system according to claim 6, further comprising:
an input interface configured to allow input of instruction information to instruct that the activation date and time be set to a date and time corresponding to either one of the recommended wake-up date and time or an arbitrary wake-up date and time,
wherein the acquisition unit acquires the instruction information that is input to the input interface, and
the setting unit sets the activation date and time on a basis of the instruction information acquired by the acquisition unit.

8. The infant wake-up assisting system according to claim 6 or 7, wherein:
the wake-up prompting device includes at least an LED, and
the wake-up prompting operation is an operation that changes the sleeping environment by initiating light emission from the LED at the activation date and time.

9. The infant wake-up assisting system according to claim 8, wherein a color temperature of light emitted from the LED, the light emission of which is initiated at the activation date and time, is greater than or equal to 4200 K and less than or equal to 6500 K.

10. The infant wake-up assisting system according to claim 9, wherein a brightness of the light emitted from the LED increases with a passage of time from the activation date and time.

11. The infant wake up assisting system according to any one of claims 1 to 10, wherein the computation unit calculates the recommended wake-up date and time on a basis of the plurality of days worth of sleeping status data, the deviations, and a predetermined target wake-up time.
